## Europäisches Patentamt

## European Patent Office

## Office européen des brevets

(11) Veröffentlichungsnummer: **0 089 094**
**B1**

(12) **EUROPÄISCHE PATENTSCHRIFT**

(45) Veröffentlichungstag der Patentschrift:
04.02.87

(21) Anmeldenummer: **83200357.8**

(22) Anmeldetag: **15.03.83**

(51) Int. Cl.⁴: **A 61 B 6/06, G 03 B 42/02**

(54) **Röntgenzielgerät mit einer Einrichtung zur filmnahen Einblendung.**

(30) Priorität: **17.03.82 DE 3209683**

(43) Veröffentlichungstag der Anmeldung:
**21.09.83 Patentblatt 83/38**

(45) Bekanntmachung des Hinweises auf die Patenterteilung:
**04.02.87 Patentblatt 87/6**

(84) Benannte Vertragsstaaten:
**BE DE FR GB**

(56) Entgegenhaltungen:
**DD-A-109 295**
**DE-B-2 424 619**
**DE-C-2 842 659**

(73) Patentinhaber: **Philips Patentverwaltung GmbH,
Billstrasse 80, D-2000 Hamburg 28 (DE)**
(84) Benannte Vertragsstaaten: **DE**

(73) Patentinhaber: **N.V. Philips' Gloeilampenfabrieken,
Groenewoudseweg 1, NL- 5621 BA Eindhoven (NL)**
(84) Benannte Vertragsstaaten: **BE FR GB**

(72) Erfinder: **Schwieker, Horst- Hartwig, Trenknerweg
21, D-2000 Hamburg 52 (DE)**
Erfinder: **Gieschen, Kurt, Kielkoppelstrasse 82i,
D-2000 Hamburg 73 (DE)**

(74) Vertreter: **Hartmann, Heinrich, Dipl.- Ing., Philips
Patentverwaltung GmbH Billstrasse 80 Postfach
10 51 49, D-2000 Hamburg 28 (DE)**

## Beschreibung

Die Erfindung bezieht sich auf ein Röntgenzielgerät mit einer Einrichtung zur filmnahen Einblendung, die eine mit symmetrisch zur horizontalen Mittellinie verlaufenden horizontalen Blendenkanten und eine weitere Blendenanordnung mit vertikal verlaufenden Blendenkanten umfaßt.

Ein solches Zielgerät ist aus der DE-PS 28 42 659 bekannt. Die Begriffe "horizontal" und "vertikal" beziehen sich dabei auf das stehende Zielgerät, wobei sich die beiden Blendenanordnungen in vertikalen Ebenen erstrecken. Als "Mittellinie" wird eine (horizontale oder vertikale) Gerade bezeichnet, die durch das Zentrum des von Strahlung getroffenen Aufnahme- oder Durchleuchtungsfeldes verläuft.

Bei Fig. 4 der bekannten Einrichtung besteht die Blende aus einer U-förmigen Blendenplatte und die weitere Blendenanordnung aus einer rechteckigen Blendenplatte. Bei einer vertikalen Unterteilung werden die beiden Blendenplatten so aufeinander zugeschoben, daß zwischen den einander zugewandten vertikalen Rändern ein entsprechend großes Aufnahmefeld verbleibt. Bei einer vertikalen und horizontalen Unterteilung einer Aufnahme werden die beiden Blendenplatten so aneinander vorbeigeschoben, daß das Aufnahmefeld durch das horizontal liegende U der ersten Blendenplatte und die andere vertikale Blendenkante der zweiten Blendenplatte begrenzt wird.

Ein Nachteil des bekannten Röntgenzielgerätes besteht darin, daß bei einer vertikalen Unterteilung das Aufnahmefeld eine vorgegebene Höhe hat. Außerdem hängt die Steuerung jeder der beiden Blendenplatten sowohl von der Unterteilung in horizontaler Richtung als auch von der Unterteilung in vertikaler Richtung ab.

Es sind außerdem Zielgeräte bekannt, bei denen zur Unterteilung des Filmformates in der Breite, d.h. zur vertikalen Unterteilung, zwei Blendenplatten vorgesehen sind, die miteinander so gekoppelt sind, daß ihre einander zugewandten vertikalen Kanten stets synchron zur vertikalen Mittellinie verlaufen. Für Aufnahmen, bei denen das Filmformat zusätzlich in der Höhe, d.h. vertikal, unterteilt werden soll, können auswechselbare Platten oder Kompressionstuben in den Strahlengang gebracht werden. Das Auswechseln stört jedoch den Untersuchungsverlauf und ist bei verschiedenen Untersuchungsarten nicht möglich.

Es ist Aufgabe der vorliegenden Erfindung, ein Röntgenzielgerät der eingangs genannten Art so auszugestalten, daß bei vertikaler Unterteilung des Aufnahmeformates die Einblendung unterschiedlichen Aufnahmeformaten angepaßt werden kann.

Diese Aufgabe wird erfindungsgemäß durch die im Kennzeichen des Hauptanspruches angegebenen Maßnahmen gelöst. Bei der Erfindung kann die filmnahe Einblendung unterschiedlich hohen Aufnahmefeldern dadurch angepaßt werden, daß die Blende so weit in den Strahlengang geschoben wird, bis derjenige Ausschnitt die vertikalen Abmessungen des Aufnahmefeldes bestimmt, dessen Blendenkanten einen der gewünschten Höhe entsprechenden Abstand haben. Die Breite der Einblendung wird dabei durch den Abstand der einander zugewandten Blendenkanten der beiden Blendenplatten der weiteren Blendenanordnung bestimmt. Die Position der Blende bestimmt die Höhe der Einblendung, und die Positionen der beiden Blendenplatten der weiteren Blendenanordnung bestimmen die Breite der Einblendung.

Die Erfindung wird nachstehend anhand der Zeichnung näher erläutert. Es zeigen:

Fig. 1 eine Ausführungsform der Erfindung in schematischer Darstellung,

Fig. 2 die Steuerung für die Positionierung jeweils einer der beiden Blendenanordnungen.

Die Einrichtung zur filmnahen Einblendung besteht aus der durch eine ebene Blende 1 und der durch die beiden rechteckigen Blendenplatten 2 gebildeten weiteren Blendenanordnung. Aufgabe dieser Einrichtung ist es, die nicht zu belichtenden Filmflächen gegen Streustrahlung abzuschirmen und durch Abbildung ihrer Kanten auf den Film die Ränder einer unterteilten Aufnahme festzulegen. Sie befinden sich daher im schematisch dargestellten Zielgerät 3 in zueinander parallelen Ebenen unmittelbar vor dem nicht näher dargestellten Film. Mit 4 ist das Aufnahmefeld bezeichnet. Das ist die Fläche die in der Filmebene von dem Strahlenbündel getroffen wird, und zwar ist in der Zeichnung das größtmögliche Aufnahmefeld z.B. 35 x 35 cm dargestellt. Die horizontale Mittellinie des Aufnahmefeldes ist mit 41 bezeichnet und die vertikale Mittellinie mit 42. Die u.a. durch eine Formatunterteilung möglichen kleineren Aufnahmefelder liegen stets symmetrisch zu den Mittellinien 41 und 42.

Der Abstand der einander zugewandten vertikal verlaufenden Längsseiten der beiden rechteckigen Blendenplatten 2 bestimmt die Breite des Aufnahmefeldes. Die symmetrisch zur vertikalen Mittellinie 42 angeordneten Blendenplatten 2 sind mit einem Zahnriemen 21 verbunden, der über eine Rolle 22, die links unten, und eine Rolle 23, die rechts unten am Zielgerät drehbar befestigt sind, gefürt ist, wobei die Rolle 23 durch einen Motor 24 antreibbar ist. Die linke Blendenplatte 2 ist mit dem oberhalb der Rollen verlaufenden Teil des Zahnriemens 21 verbunden, während die rechte Blendenplatte 2 mit dem Teil des Zahnriemens 21 unterhalb der Rollen 22, 23 verbunden ist. Dadurch werden die Blendenplatten 2 in horizontaler Richtung gegensinnig, jedoch stets um die gleiche Strecke aufeinander zu oder voneinander weg bewegt, je nachdem, ob die Rolle 23 rechts oder links herum vom Motor 24 angetrieben wird.

Die Blende 7, die ebenso wie die Blendenplatte 2 vorzugsweise aus Blei besteht, ist mit einem Zahnriemen 11 verbunden, der über am Zielgerät befestigten Rollen 12 und 13 geführt ist. Die Rolle 13 wird von einem Motor 14 angetrieben. Auf diese Weise kann auch die Blende 1 in horizontaler Richtung verschoben werden. Die Blende 1 besitzt drei rechteckige, zur horizontalen Mittellinie symmetrisch angeordnete Ausschnitte 15, 16 und 17, die unmittelbar ineinander übergehen, so daß zwischen ihnen kein Steg verbleibt. Der Ausschnitt 17 hat eine Höhe von z.B. 12 cm, d.h. die ihn begrenzenden horizontalen Blendenplatten verlaufen in einem Abstand von 6 cm von der horizontalen Mittellinie, und eine Breite von z.B. 15 cm. Der Ausschnitt 16 ist 9 cm hoch und 12 cm breit, während der Ausschnitt 15 etwa 4,5 cm hoch und ebenfalls etwa 12 cm breit ist. Die Kanten der Ausschnitte 15, 16 und 17 haben also oberhalb bzw. unterhalb der Mittellinie 14 eine nach rechts hin aufsteigende bzw. abfallende Treppenform. In der Zeichnung sind die drei Ausschnitte beidseitig durch das Material der Platte begrenzt, doch könnte grundsätzlich die Platte nach links oder rechts hin offen sein.

Wenn das Aufnahmeformat in der Breite, d.h. horizontal, unterteilt werden soll, wird lediglich der Motor 24 eingeschaltet und die Platten 2 so weit in den Strahlengang eingefahren, bis die gewünschte Breite eingestellt ist. Soll zusätzlich das Aufnahmeformat noch in der Höhe unterteilt werden, wird auch die Blende 1 so weit in den Strahlengang gefahren, daß der Ausschnitt, der die gewünschte Höhe hat, mit seiner (horizontalen) Mitte mit der vertikalen Mittellinie 42 zusammenfällt. Die Blendenplatten 2 müssen dabei so weit aufeinander zu bewegt werden, daß die Breite des durch sie begrenzten Feldes nicht größer ist als die Breite des entsprechenden Ausschnittes; jedoch können die Blendenplatten 2 auch noch weiter zusammengefahren werden.

Soll z.B. das Filmformat 24 x 30 cm quer (die Begriffe quer bzw. hoch bedeuten, daß die längere Seite quer bzw. hoch steht, d.h. horizontal bzw. vertikal verläuft) vierfach unterteilt werden (zweimal in der Höhe und zweimal in der Breite), dann wird die Blende 1 so weit nach links gefahren, bis die Mitte des Ausschnittes 17 mit der vertikalen Mittellinie 42 zusammenfällt. Die Blendenplatten 2 werden so weit zusammengefahren, daß ihr Abstand etwa 15 cm beträgt. Wenn stattdessen das Format 24 x 24 cm hoch vierfach unterteilt werden soll, wird die Blende 1 in die gleiche Stellung gefahren, jedoch müssen die Blendenplatten 2 dichter zusammengefahren werden, weil die Breite des Aufnahmefeldes geringer geworden ist.

Man erkennt aus diesem Beispiel, daß die Breite eines Ausschnittes 17 für die Einblendung nicht bestimmend ist. Die Breite eines Ausschnittes muß daher so gewählt sein, daß sie größer oder gleich der größten Breite des Aufnahmeformates (im gewählten Beispiel etwa 15 cm) bei der durch die vertikalen Abmessungen (beim Ausschnitt 17 12 cm) vorgegebenen Unterteilung ist. Wird die Breite größer gewählt, werden die Anforderungen an die Positionierungsgenauigkeit der Blende 1 geringer; jedoch wird dadurch der Platzbedarf und das Gewicht der Blende 1 größer.

Soll das Aufnahmeformat 18 x 24 cm quer vierfach unterteilt werden, wird die Blende 1 so weit nach links gebracht, bis die Mitte des Ausschnittes 16 mit der vertikalen Mittellinie 42 zusammenfällt und die Blenden 2 werden entsprechend zusammengefahren. Bei Colonaufnahmen, bei denen das Format 18 x 24 cm quer achtfach unterteilt werden soll (zweifach in der Breite und vierfach in der Höhe), wird der Ausschnitt 15 in die Mitte gefahren.

Es ergibt sich der Vorteil, daß Unterteilungen mit unterschiedlicher Höhe möglich sind, ohne daß eine Platte oder ein Tubus gewechselt werden muß, was bei bestimmten Untersuchungsverfahren auch nicht möglich ist.

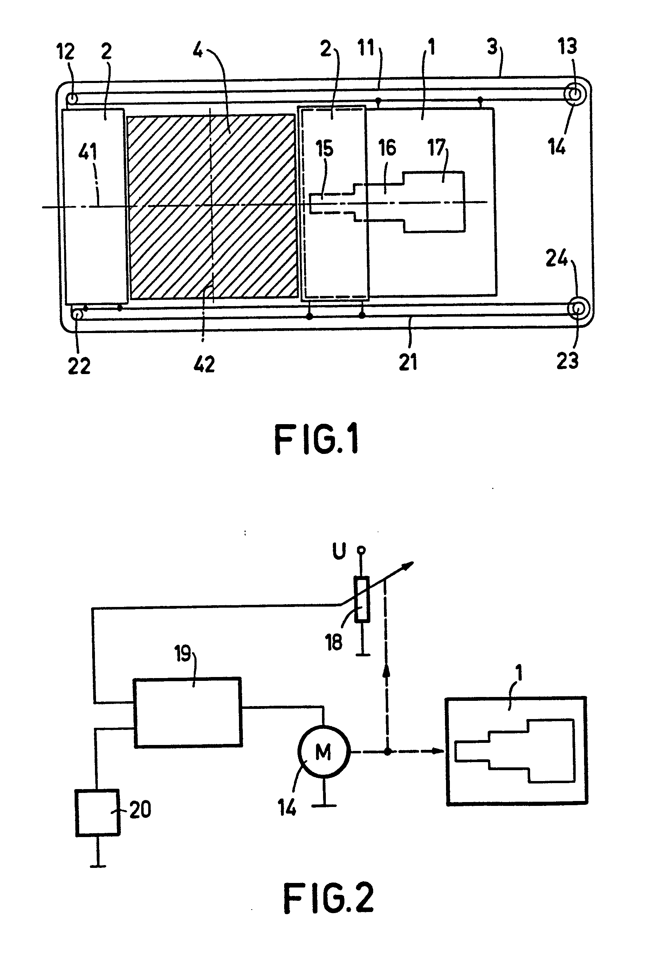

In Fig. 2 ist schematisch die Steuerschaltung für den Motor 14, der die Blende 7 verachiebt, dargestellt. Der Motor 14, der gemäß Fig. 1 über einen Zahnriemen 11 die Blende 1 in horizontaler Richtung verschiebt, verstellt gleichzeitig den z.B. mit der Rolle 13 gekoppelten Abgriff eines Potentiometers 18, das an einer Spannung U angeschlossen ist. Die Spannung am Abgriff 18 ist daher ein Maß für die jeweilige Position der Blende 1. In einem Regelverstärker 19 wird die dem Postions-Istwert entsprechende Spannung am Abgriff des Potentiometers 18 mit einem Positions-Sollwert verglichen, der in Form einer Gleichspannung von einem Sollwert-Geber 20 geliefert wird. Der Sollwert-Geber 20 kann ein geeigneter Widerstands-Spannungsteiler sein. Grundsätzlich kann der Sollwert jedoch auch von einem MIkroprozessor über einen Digital-Analog-Wandler geliefert werden. Der Motor 24 wird entsprechend gesteuert, wie an sich auch aus der DE-OS 22 48 101 bekannt ist.

**Patentansprüche**

1. Röntgenzielgerät mit einer Einrichtung zur filmnahen Einblendung, die eine Blende (1) mit symmetrisch zur horizontalen Mittellinie (41) verlaufenden horizontalen Blendenkanten und eine weitere Blendenanordnung (2, 21, 22, 23) mit vertikal verlaufenden Blendenkanten umfaßt, dadurch gekennzeichnet, daß die Blende (1) mehrere in horizontaler Richtung gegeneinander versetzte, zur horizontalen Mittellinie (41) symmetrische, rechteckförmige Ausschnitte (15, 16, 17) mit voneinander abweichenden vertikalen Abmessungen aufweist, und daß die weitere Blendenanordnung aus zwei Blendenplatten (2) besteht, die in horizontaler Richtung gegensinnig zueinander bewegbar sind, so daß ihre einander zugewandten vertikalen Kanten stets symmetrisch zur vertikalen Mittellinie (42) verlaufen.

2. Röntgenzielgerät nach Anspruch 1, <u>dadurch gekennzeichnet</u>, daß benachbarte Ausschnitte (z.B. 15, 16) ohne vertikale Begrenzung ineinander übergehen.

3. Röntgenzielgerät nach Anspruch 1, <u>dadurch gekennzeichnet</u>, daß die horizontalen Abmessungen jedes Ausschnittes (15, 16, 17) ungefähr so groß sind wie die größte Breite des Aufnahmefeldes bei einer durch seine vertikalen Abmessungen bestimmten Unterteilung.

## Claims

1. An X-ray target apparatus with a fade-in device close to the film, comprising a diaphragm (1) with horizontal diaphragm edges extending symmetrically to the horizontal centre line (41) and a further diaphragm assembly (2, 21, 22, 23) with vertically extending diaphragm edges, characterized in that the diaphragm (1) comprises several rectangular syctions (15, 16, 17) which are relatively displaced in the horizontal direction, which extend symmetrically to the horizontal centre line (41) and which have relatively different vertical dimensions, the further diaphragm assembly consisting of two diaphragm plates (2) which are displaceable in the opposite sense relative to one another in the horizontal direction, so that their vertical edges facing each other invariably extend symmetrically to a vertical centre line (42).

2. An X-ray target apparatus as claimed in claim 1, characterized in that adjacent sections (for example 15, 16) join each other without vertical limitation.

3. An X-ray target apparatus as claimed in Claim 1, characterized in that the horizontal dimensions of each of the sections (15, 16, 17) approximately equal the largest width of the exposure field with a sub-division determined by its vertical dimensions.

## Revendications

1.- Appareil à cible radiographique pourvu d'un dispositif de masquage opérant près du film, qui comporte un écran (1) présentant des bords d'écran horizontaux qui s'étendent symétriquement à l'axe médian horizontal (41) et un autre ensemble d'écran (2, 21, 22, 23) présentant des bords d'écran qui s'étendent verticalement, caractérisé en ce que l'écran (1) présente plusieurs découpes rectangulaires décalées l'une de l'autre dans le sens horizontal, symétriques par rapport à l'axe médian horizontal (41) et de dimensions verticales différentes et l'autre ensemble d'écran est formé de deux plaques d'écran (2) qui peuvent être déplacées en sens opposés dans la direction horizontale de sorte que leurs bords verticaux tournés l'un vers l'autre s'étendent toujours symétriquement par rapport à l'axe médian vertical (42).

2.- Appareil à cible radiographique suivant la revendication 1, caractérisé en ce que des découpes voisines (par exemple 15, 16) se raccordent l'une à l'autre sans délimitation verticale.

3.- Appareil à cible radiographique suivant la revendication 1, caractérisé en ce que les dimensions horizontales de chaque découpe (15, 16 et 17) sont environ égales à la plus grande largeur du champ radiographique dans le cas d'une subdivision déterminée par ses dimensions verticales.

FIG.1

FIG.2